# EUROPEAN PATENT SPECIFICATION

(11) **EP 2 610 212 B1**
(45) Date of publication and mention of the grant of the patent: **01.06.2016**
(21) Application number: 11819626.0
(22) Date of filing: 15.03.2011
(51) Int. Cl.: B82B 1/00, B82B 3/00, C09K 11/08, C09K 11/54, C09K 11/56, C09K 11/88

(54) **SEMICONDUCTOR NANOPARTICLE AGGREGATE AND PRODUCTION METHOD FOR SEMICONDUCTOR NANOPARTICLE AGGREGATE**
HALBLEITER-NANOPARTIKEL-AGGREGAT UND HERSTELLUNGSVERFAHREN FÜR EIN HALBLEITER-NANOPARTIKEL-AGGREGAT
AGRÉGAT DE NANOPARTICULES SEMI-CONDUCTRICES ET PROCÉDÉ DE FABRICATION D'UN AGRÉGAT DE NANOPARTICULES SEMI-CONDUCTRICES

(30) Priority: 27.08.2010 JP 2010190354
(43) Date of publication of application: 03.07.2013
(73) Proprietor: Konica Minolta, Inc., Tokyo 100-7015 (JP)
(72) Inventor: HOSHINO, Hideki, Hino-shi Tokyo 191-8511 (JP); TAKAHASHI, Masaru, Hino-shi Tokyo 191-8511 (JP); GONDA, Kohsuke, Sendai-shi Miyagi 980-8577 (JP); TAKEDA, Motohiro, Sendai-shi Miyagi 980-8577 (JP); OHUCHI, Noriaki, Sendai-shi Miyagi 980-8577 (JP)
(74) Representative: Gille Hrabal
(86) International application number: PCT/JP2011/056000
(87) International publication number: WO 2012/026150

(56) References cited:
- EP-B1- 1 797 224
- WO-A1-2009/028390
- WO-A1-2009/028390
- WO-A1-2010/007956
- JP-A- 2009 281 760
- US-A1- 2008 230 750
- NORIKO EIHA, ET AL.: "Collective Fluorescence Oscillation in a Water Dispersion of Colloidal Quantum Dots", JAPANESE JOURNAL OF APPLIED PHYSICS, PART 2, vol. 42, no. 3B, 15 March 2003 (2003-03-15), pages L310-L313, XP002720963, JAPAN SOC. APPL. PHYS. JAPAN ISSN: 0021-4922, DOI: 10.1143/JJAP.42.L310
- JUN MIYAZAKI ET AL.: 'Excited state dynamics of CdSe/CdZnS quantum dots in solution' ABSTRACTS OF THE MEETING OF THE PHYSICAL SOCIETY OF JAPAN vol. 64, no. 2, 18 August 2009, page 276, XP008167965

## Description

### [Technical field]

The present invention relates to a semiconductor nanoparticle aggregate having high light emission luminance and small variation in luminance per particle, and a production method for the semiconductor nanoparticle aggregate.

### [Background art]

When fluorescence semiconductor nanoparticles are used as a labeling agent, the semiconductor nanoparticles with higher luminance per particle have a higher sensitivity, so particles having higher luminance per particle are desired. Also, the smaller the variation in luminance per particle of a labeling agent is, the more possible it is to conduct quantitative assessment, and particles having smaller variation in luminance per particle are thus desired.

As fluorescence semiconductor nanoparticles, II-VI and III-V semiconductor nanoparticles are widely known. When it comes to use these semiconductor nanoparticles as a fluorescence diagnostic agent, luminance per particle thereof is still far insufficient.

Meanwhile, generally, when only core semiconductor nanoparticles are used, luminance of the particles is much lower than that of semiconductor nanoparticles having a core/shell structure. By using a semiconductor material having a wider bandgap than that of core particles as a shell, quantum wells are formed, and luminance is remarkably improved due to a quantum containment effect

Therefore, as a method of improving luminance, a method is considered in which core/shell semiconductor nanoparticles are aggregated to improve luminance per particle.

For example, a technique has been proposed, in which semiconductor nanoparticles are contained in a dispersed and fixed fashion in a matrix of transparent glass or the like, so that a solid-state material is obtained, which exerts a high-luminance light emitting property over a long period of time under various environments and is suitable for technological application.

Patent Literature 1 discloses a glass phosphor, in which semiconductor nanoparticles are dispersed and fixed inside thereof by combining a reverse-micelle method, and a sol-gel method where a mixture of organic alkoxysilane and alkoxide is used as a glass precursor, the organic alkoxysilane having an organic functional group, which is well adsorbed onto semiconductor nanoparticles, in ends of molecules thereof. However, reduction in luminance efficiency is observed due to an influence of a reaction in the sol-gel method. Further, since hydrolyzed products of organic alkoxysilane and alkoxide are contained, there is a problem that a distance between the semiconductor nanoparticles is extended, and it is thus impossible to collect a specific amount of semiconductor particles in high concentration, resulting in insufficient luminance.

Furthermore, in the sol-gel method, it is difficult to control particle sizes of the glass phosphor obtained, causing large variation in luminance per particle. Therefore, particles with a small variation coefficient of particle size are desired.

### [Prior Art Documents]

### [Patent literature]

[PTL 1] Japanese Patent Application Laid-open Publication No. 2005-281019

US 2008/230750 A discloses semiconductor nanoparticle aggregates containing semiconductor nanoparticles with a core/shell structure, wherein CdSe, CdTe and InP are used as core parts and ZnS is used a shell part.

EP 1 797 224 B discloses semiconductor nanoparticte aggregates containing semiconductor nanoparticles containing a coating layer. The coating layer comprises imidazole groups and CdSe. CdTe, InP and ZnS are used core and/or shell parts.

WO 2009/028390 A discloses semiconductor nanoparticle aggregates containing semiconductor nanoparticles with a core/shell structure, wherein CdSe, CdTe and InP are used as core parts and ZnS is used a shell part.

Japanese Journal of applied physics, Part 2 (Letters), 42, 3B, pages L310 to L313 discloses semiconductor nanoparticle aggregates containing semiconductor nanoparticles with a core/shell structure, wherein CdSe, CdTe and InP are used as core and shell parts.

### [Summary of the invention]

### [Problems to be solved by the invention]

The present invention has been accomplished in view of the above-mentioned problems and situations, and a problem to be solved is to provide a semiconductor nanoparticle aggregate having high light emission luminance and a small variation coefficient of particle size, in which fluorescence semiconductor nanoparticles are aggregated densely, and a production method for the semiconductor nanoparticle aggregate.

### [Means for solving the problems]

According to the invention set forth in claim 1, provided is a semiconductor nanoparticle aggregate containing semiconductor nanoparticles having a core/shell structure, wherein an agglomeration state of an agglomerate made by agglomerating the semiconductor nanoparticles is controlled by using physical energy, thereby forming the semiconductor nanoparticle aggregate; wherein a coating structure is formed so as to coat the agglomerate made by agglomerating the semiconductor nanoparticles, thereby forming the semiconductor nanoparticle aggregate; wherein a raw material that configures the coating structure is hydrophilic polymer; wherein an average particle size of the nanoparticle aggregate is in a range from 30 to 300 nm; characterized in that a variation coefficient of particle size of the nanoparticle aggregate is in a range from 0.02 to 0.2 and wherein the particle sizes of the semiconductor nanoparticle aggregates are measured by a dynamic light scattering method.

According to the invention set forth in claim 2, provided the raw material that forms the coating structure is polyvinyl alcohol.

According to the invention set forth in claim 3, a raw material that forms a shell part of the semiconductor nanoparticle having the core/shell structure is zinc sulfide (ZnS) or silicon dioxide (SiO₂).

According to the invention set forth in claim 4, the raw material that forms a core part of the semiconductor nanoparticle having the core/shell structure is selected from Si, Ge, InN, InP, GaAs, AlSe, CdSe, AlAs, GaP, ZnTe, CdTe, and InAs.

According to the invention set forth in claim 5, further comprising a surface modifier on a surface of the semiconductor nanoparticle aggregate, wherein the surface modifier is mercaptopropionic acid, mercaptoundecanoic acid, or aminopropanethiol.

According to the invention set forth in claim 6, a disperser is used to supply the physical energy and an amount of physical energy is 20 MPa to 300 MPa.

According to the invention set forth in claim 7, the amount of physical energy is 70 MPa to 150 MPa.

According to the invention set forth in claim 8 a production method for a semiconductor nanoparticle aggregate containing semiconductor nanoparticles having a core/shell structure is claimed, comprising: controlling by using physical energy an agglomeration state of an agglomerate made by agglomerating the semiconductor nanoparticles, thereby forming the semiconductor nanoparticle aggregate; wherein a coating structure is formed so as to coat the agglomerate made by agglomerating the semiconductor nanoparticles, thereby forming the semiconductor nanoparticle aggregate; wherein a raw material that configures the coating structure is hydrophilic polymer; wherein an average particle size of the nanoparticle aggregate is in a range from 30 to 300 nm; characterized in that a variation coefficient of particle size of the nanoparticle aggregate is in a range from 0.02 to 0.2 and wherein the particle sizes of the semiconductor nanoparticle aggregates are measured by a dynamic light scattering method.

According to the present invention, by controlling an agglomeration state of an agglomerate of fluorescence semiconductor, it becomes possible to provide a semiconductor nanoparticle aggregate having high light emission luminance, a small variation of luminance per particle, and a small variation coefficient of particle size.

### [Best modes for carrying out the invention]

A semiconductor nanoparticle aggregate according to the present invention will be described below.

### (Semiconductor nanoparticle aggregate)

In the present application, "semiconductor nanoparticles, having a core/shell structure" mean nanosize particles (1 to 20 nm) that contain a semiconductor forming material (raw material) described later, and have a multi-layer structure including a core part and a shell part that covers the core part.

In the present application, a "semiconductor nanoparticle aggregate" means a particle that has a structure in which a plurality of core/shell semiconductor nanoparticles is aggregated. The aggregate means a form that is obtained by controlling an agglomeration state of an agglomerate of the above-mentioned semiconductor nanoparticles using physical energy, and the aggregate is different from the agglomerate in which particles are densely gathered in an unspecified manner by a secondary force between molecules, for example.

### (Production method for semiconductor nanoparticle aggregate)

As a production method for the semiconductor nanoparticle aggregate according to the present invention, a method using a liquid phase method or a gas phase method may be adopted. The liquid phase method is particularly favorable in terms of reaction controllability and manufacturing costs.

A production method for the semiconductor nanoparticle aggregate using the liquid phase method will be explained below. In a formation process of semiconductor nanoparticles using a later-described production method for the semiconductor nanoparticles or in a dispersion process after the semiconductor nanoparticles are formed, the semiconductor nanoparticle aggregate is formed by using a poor solvent in which the semiconductor nanoparticles are hardly dissolved.

Here, as the poor solvent, alcohols (methanol, ethanol, propanol, and so on) and ketones (acetone, methyl ethyl ketone, and so on) may be used. An agglomeration state of an agglomerate of the semiconductor nanoparticles agglomerated by the poor solvent is controlled as a given physical energy is applied to the agglomerate, thus forming the semiconductor nanoparticle aggregate.

Here, examples of the physical energy include ultrasonic waves, a colliding force, and a shear force, and the energy is able to be applied by using various types of dispersers. An amount of the energy used is not particularly limited and may be adjusted where necessary so as to obtain a desired particle size and variation coefficient. However, an excessively large amount of energy results in braking up of the semiconductor nanoparticles themselves and polydispersion due to overdispersion. Therefore, when a disperser in which, for example, pressure is used as the physical energy, a favorable amount of energy ranges from 20 MPa to 300 MPa, and a range from 70 MPa to 150 MPa is preferably applied as more favorable amount of energy. The reason why the amount of energy is in the range from 20 MPa to 300 MPa is because pressure less than 20 MPa is not sufficient as energy for controlling an agglomeration state, and it thus becomes impossible to polydisperse the obtained semiconductor nanoparticle aggregate and disperse the same to a desired particle size. A pressure over 300 MPa causes breaking up of the semiconductor nanoparticles themselves, fluorescence property is thereby inactivated, and desired semiconductor nanoparticle aggregate is not able to be obtained.

In the formation process of the semiconductor nanoparticles, or in the dispersion process after the formation of the semiconductor nanoparticles, a raw material for forming a coating structure is added so that the semiconductor nanoparticle aggregate is coated, thus forming a semiconductor nanoparticle aggregate having the coating structure.

### <Material for forming a coating structure>

In the present invention, the raw material for forming the coating structure is not particularly limited, and any organic and inorganic substances may be applied depending on the intended use as long as the substance is able to coat the semiconductor nanoparticle agglomerate. For example, in a case where the semiconductor nanoparticle aggregate according to the present invention is used as a later-described biological substance labeling agent, when a surface of the semiconductor nanoparticle aggregate is hydrophobic, water dispersibility thereof is bad, thus causing a problem that the semiconductor nanoparticle aggregates are agglomerated, so it is preferred to hydrophilize the surface of the semiconductor nanoparticle aggregate. Examples of a raw material for hydrophiliation include silica and hydrophilic polymer (gelatin, guar gum, carboxymethyl cellulose, pectin, karaya gum, polyvinyl alcohol, and so on). Among the hydrophilic polymers, polyvinyl alcohol is particularly favorable in terms of handling and lot stability as polyvinyl alcohol is a synthetic material.

### <Particle size of semiconductor nanoparticle aggregate and numbers of particles included therein>

In the present invention, it is preferred that an average particle size of the semiconductor nanoparticle aggregates is in a range from 5 to 1000 nm, and more preferably in a range from 30 to 300 nm. The reason why the average particle size is in the range from 5 to 1000 nm is because an average particle size of less than 5 nm is almost equal to a particle size of a single semiconductor nanoparticle, and an average particle size over 1000 nm stops improvement of fluorescence intensity according to the number of aggregated semiconductor nanoparticles, for some unknown reasons.

Particle sizes of the core/shell semiconductor nanoparticles and the semiconductor nanoparticle aggregates are able to be measured by a dynamic light scattering method.

The number of semiconductor nanoparticles gathered (included) is calculated as follows. First, an element ratio of the semiconductor nanoparticles is measured by using ICP-AES (ICPS-7500 produced Shimadzu Corporation). Thereafter, an element ratio of the semiconductor nanoparticle aggregates is measured by using ICP-AES, thus calculating a concentration. Since densities of the semiconductor nanoparticles and semiconductor nanoparticle aggregates are already known, the number semiconductor nanoparticles gathered is able to be estimated based on the densities as well as the average particle sizes calculated as above in the dynamic light scattering method.

### <Variation coefficient of semiconductor nanoparticles>

In the present invention, it is preferred that a variation coefficient of a particle size distribution of the semiconductor nanoparticles is in a range from 0.02 to 0.2. A range from 0.03 to 0.1 is further preferred. The reason why the variation coefficient is in a range from 0.02 to 0.2 is because a variation coefficient above 0.2 means a volume of a semiconductor nanoparticle aggregate having a maximum particle size becomes at least twice as large as the volume of a particle having an average particle size, and variation in fluorescent luminance per semiconductor nanoparticle aggregate becomes large. On the contrary, fabricating the semiconductor nanoparticle aggregates having a variation coefficient of less than 0.02 requires enormous costs but does not show significant difference in the properties.

### <Production method for semiconductor nanoparticles>

A method using a liquid phase method or a gas phase method is applied as a production method for semiconductor nanoparticles according to the present invention.

Examples of a production method using the liquid phase method include a precipitation method, a coprecipitation method, a sol-gel method, a homogeneous precipitation method, and a reduction method. In addition, a reverse-micelle method, and a supercritical hydrothermal synthesis method are also excellent methods for fabricating nanoparticles (see Japanese Patent Application Laid-open Publication No. 2002-322468, Japanese Patent Application Laid-open Publication No. 2005-239775, Japanese Patent Application Laid-open Publication No. 10-310770, and Japanese Patent Application Laid-open Publication No. 2000-104058).

In a case where the semiconductor nanoparticle agglomerates are produced by using the liquid phase method, it is also preferred that the production method includes a process in which a precursor of the semiconductor is reduced by reduction reaction.

Further, it is preferred that the production method includes a process in which the precursor of the semiconductor is reacted in the presence of a surface acting agent. The semiconductor precursor according to the present invention is a chemical compound containing elements that are used as the above-mentioned semiconductor material, and, when, for example, the semiconductor is Si, the semiconductor precursor may be SiCl4 or the like. Other examples of the semiconductor precursor include InC13, P(SiMe3)3, ZnMe2, CdMe2, GeC14, and selenium-tributylphosphine.

A reaction temperature of a reaction precursor is not particularly limited as long as it is a boiling point of the semiconductor precursor or higher, and a boiling point of a solvent or lower, but a range from 70 to 110 °C is preferred.

### <Raw material for forming a core part of a semiconductor nanoparticle>

As a raw material for forming a core part (also referred to as a "core particle"), semiconductors such as Si, Ge, InN, InP, GaAs, AlSe, CdSe, AlAs, GaP, ZnTe, CdTe, and InAs, or materials forming such semiconductors may be used. In the present invention, InP, CdTe, and CdSe are particularly preferred.

### <Raw material for forming a shell part of a semiconductor nanoparticle>

As a raw material for forming a shell part, II-VI, III-V, or IV inorganic semiconductor may be used. A preferred example includes Si, Ge, InN, InP, GaAs, AlSe, CdSe, AlAs, GaP, ZnTe, CdTe, and InAs, which are non-toxic semiconductor having a larger bandgap than that of each of the inorganic materials for forming a core part, or a material for forming such semiconductor. More preferably, ZnS is used as a shell for InP, CdTe, or CdSe, and SiO2 is used as a shell for Si.

### <Reductant>

As a reductant for reducing the semiconductor precursor, various types of conventionally-known reductants may be used depending on reaction conditions. In the present invention, from the viewpoint of a level of reduction ability, reductants such as lithium aluminum hydride (LiAlH4), sodium boron hydride (NaBH4), sodium bis (2-methoxyethoxy) aluminum dihydride, lithium tri(sec-butyl)borohydride (LiBH(sec-C4H9)3), potassium tri(sec-butyl)borohydride, and lithium triethylborohydride are preferred. In particular, lithium aluminum hydride (LiAlH4) is preferred because of the level of the reduction ability thereof.

### <Solvent>

Although various types of conventionally-known solvents may be used as a solvent for dispersion of a semiconductor precursor, it is preferred to use alcohols such as ethyl alcohol, sec-butyl alcohol, and t-butyl alcohol, and solvents of hydrocarbons such as toluene, decane, and hexane. In the present invention, a hydrophobic solvent such as toluene is particularly preferred as a solvent for dispersion.

### <Surface active agent>

Various types of conventionally-known surface active agents, such as anionic, non-ionic, cationic, and ampholytic surface active agents may be used as a surface active agent. In particular, a quaternary ammonium chloride group including tetrabutylammonium chloride, bromide or hexafluorophosphate, tetraoctylammonium bromide (TOAB), or tributylhexadecylphosphonium bromide is preferred. Tetraoctylammonium bromide is particularly preferred.

Reaction in the liquid phase method changes greatly depending on a state of a chemical compound in a liquid, including a solvent. Extra attention is required especially when fabricating nanosize particles having excellent monodispersity. For example, in the reverse-micelle method, size and state of reverse-micelle which serves as a reaction field are changed depending on a concentration or a type of a surface active agent, and conditions for forming nanoparticles are thus limited. Therefore, an adequate surface active agent needs to be combined with a solvent.

Examples of a production method using the liquid phase method includes (1) a method in which raw material semiconductor that faces each other is vaporized by a first high-temperature plasma generated between electrodes, and passed through a second high-temperature plasma generated by electrodeless discharge in a reduced-pressure atmosphere (for example, see Japanese Patent Application Laid-open Publication No. 6-279015), (2) a method in which nanoparticles are separated and removed from a positive electrode made of a raw material semiconductor, by electrochemical etching (for example, see Published Japanese Translation of PCT application No. 2003-515459), (3) a laser ablation method (for example, see Japanese Patent Application Laid-open Publication No. 2004-356163), and (4) a high-rate sputtering method (see Japanese Patent Application Laid-open Publication No. 2004-296781). It is also preferred to use a method in which a raw material gas is reacted in a gas phase in a low-pressure state, and powder containing particles is synthesized.

Although the production method for the semiconductor nanoparticle aggregate was outlined above, a specific method will be detailed in descriptions of examples.

### (Applications)

Typical applications of the semiconductor nanoparticle aggregate according to the present invention will be described below.

### <Biological substance labeling agent and bio imaging>

The semiconductor nanoparticle aggregate is applicable to a biological substance labeling agent. By adding a biological substance labeling agent to living cells or a living body having a target substance (tracer), the biological substance labeling agent is bound together with or adsorbed to the target substance, a resultant conjugate or an adsorbent is irradiated with excitation light having a given wavelength, and fluorescence with a given wavelength generated from the fluorescent semiconductor nanoparticles in accordance with the excitation light is detected, thereby performing fluorescent dynamic imaging of the above-mentioned target substance (tracer). In other words, the biological substance labeling agent is able to be used for a bio imaging method (a technological method for visualizing biomolecules included in biological substances, and dynamic phenomena of the biomolecules).

### «Hydrophilizaion treatment of semiconductor nanoparticle aggregate»

Since a surface of the foregoing semiconductor nanoparticle aggregate is generally hydrophobic, when the semiconductor nanoparticle aggregate is used as, for example, a biological substance labeling agent, dispersibility of the semiconductor nanoparticle aggregate in water is poor as it is, thus causing a problem that the semiconductor nanoparticle aggregates are agglomerated. Therefore, it is preferred to perform hydrophilization treatment on the surface of the semiconductor nanoparticle aggregate.

As an example of the hydrophilization treatment method, there is a method in which a lipophilic group on the surface of the semiconductor nanoparticle aggregate is removed by using pyridine or the like, and then a surface modifier is chemically and/or physically bound with the surface of the semiconductor nanoparticle aggregate. Preferred examples of the surface modifier are those having a carboxylic group or an amino group as a hydrophilic group, and specific examples include mercaptopropionic acid, mercaptoundecanoic acid, and aminopropanethiol. To be specific, for example, 10-5 g of Ge/GeO2 type nanoparticles is dispersed in 10 ml of pure water in which 0.2 g of mercaptoundecanoic acid is dissolved, and a resultant solution is agitated for 10 minutes at 40 °C to treat the surface of the shell, thereby modifying the surface of the shell of the inorganic nanoparticle with a carboxylic group.

### «Biological substance labeling agent»

A biological substance labeling agent is obtained by binding the foregoing hydrophilized semiconductor nanoparticle aggregate and a molecular labeling substance together through an organic molecule.

### <<Molecular labeling substance»

A biological substance labeling agent is specifically bound and/or reacts with a biological substance targeted by a molecular labeling substance, and is thus able to label the biological substance.

Examples of the molecular labeling substance include a nucleotide chain, an antibody, antigen, and cyclodextrin.

### «Organic molecule»

In a biological substance labeling agent, the hydrophilized semiconductor nanoparticle aggregate and the molecular labeling substance are bound together by an organic molecule. The organic molecule is not particularly limited as long as the organic molecule is able to bind the semiconductor nanoparticle aggregate and the molecular labeling substance, but proteins, especially albumin, myoglobin, casein, and so on, are preferably used, or avidin, which is a type of protein, may be preferably used with biotin. A form of the binding includes, but not limited to, covalent binding, ion binding, hydrogen binding, coordination binding, physical adsorption, and chemical adsorption. In terms of binding stability, binding having high binding strength, such as covalent binding, is preferred.

To be specific, in a case where the semiconductor nanoparticle aggregate is hydrophilized with mercaptoundecanoic acid, avidin and biotin are able to be used as the organic molecules. In this case, the carboxylic group of the hydrophilized nanoparticle is well bound with avidin covalently, avidin is further bound with biotin selectively, and biotin is further bound with the biological substance labeling agent, thereby forming the biological substance labeling agent.

### [Examples]

Although the present invention will be explained in more detail in the examples below, the present invention is not limited to the examples.

### 1. [Example 1]

### (1) Synthesis of InP/ZnS core/shell semiconductor nanoparticles

InP/ZnS core shell particles were synthesized by using a heating solution method described below.

6 ml of octadecene was put in a three neck flask, and, in the solvent, In(acac)3 and tris(trimethylsilyl)phosphine dissolved in 1 ml of octadecene were added so that a ratio between In and P, In/P became 1/1, and then reacted for one hour at 300 °C in an argon atmosphere. Thus, InP core particle (dispersion liquid) was obtained. Then, after an hour of reaction at 300 °C, the InP core particle dispersion solution is cooled down to 80 °C, and then zinc stearate + sulfur dissolved in 1 ml of octadecene were added in the dispersion liquid so that a ratio among In, P, Zn, and S, that is In/P/Zn/S, became 1/1/1/1. A temperature of a resultant liquid was increased from 80 °C to 230 °C to cause reaction for 30 minutes, and InP/ZnS core/shell semiconductor nanoparticles were thus obtained. The InP/ZnS core/shell semiconductor nanoparticles obtained in this way had a maximal emission wavelength of 630 nm.

### (2) Formation of semiconductor nanoparticle agglomerate

The aforementioned dispersion liquid after the reaction was cooled down to a room temperature, and then a 10-fold molar quantity of ethanol serving as a poor solvent relative to the InP/ZnS core/shell semiconductor nanoparticles was added to the dispersion liquid. Thus, an InP/ZnS agglomerates were obtained.

### (3) Formation of semiconductor nanoparticle aggregate

After repeating centrifugation and ethanol washing of a suspension containing the semiconductor nanoparticle agglomerates for several times, the suspension was replaced with pure water, and an aqueous suspension containing the semiconductor nanoparticle agglomerates were obtained.

After 5 ml of the aqueous suspension went through dispersion for three passes at 150 MPa using a wet type atomizer (Nanomizer produced by Yoshida Kikai Co., Ltd.), 0.5 ml of 10% polyvinyl alcohol aqueous solution and 0.1 ml of 0.5% sodium dodecyl sulfate aqueous solution were added to the aqueous suspension, and dispersion thereof was conducted for another four passes.

After a supernatant liquid was removed by centrifugation, a resultant liquid was replaced with pure water, put in the disperser again, and went through dispersion for two passes at 110 Mpa. Thereafter, 0.1 ml of 0.5% sodium tetraborate decahydrate was added, and a resultant liquid was dispersed for another three passes. Thus, a dispersion liquid of the semiconductor nanoparticle aggregates was formed. The dispersion liquid obtained was measured by using a particle size distribution measuring device (Zetasizer Nano produced by Malvern Instruments Ltd.), and it was found that the semiconductor nanoparticle aggregates had an average particle size of 80 nm and a variation coefficient of 0.07.

### 2. [Example 2]

### (3) Formation of semiconductor nanoparticle aggregate

After 5 ml of an aqueous suspension containing the semiconductor nanoparticle agglomerates obtained in Example 1 was dispersed for three passes at 150 MPa by using a wet type atomizer (Nanomizer produced by Yoshida Kikai Co., Ltd.), 0.02 ml of mercaptopropyltrimethoxysilane was added thereto and dispersion thereof was conducted for another two passes.

After a supernatant liquid was removed by centrifugation, a resultant liquid was replaced with pure water and ethanol at a ratio between pure water and ethanol of 1:9, put in the disperser again, and dispersed for two passes at 110 Mpa. Thereafter, 0.5 ml of tetraethoxysilane and 0.1 ml of 28% ammonia aqueous solution were added and dispersion was conducted for another three passes. Thus, a dispersion liquid of the semiconductor nanoparticle aggregates was formed. The dispersion liquid obtained was measured by using a particle size distribution measuring device (Zetasizer Nano produced by Malvern Instruments Ltd.), and it was found that the semiconductor nanoparticle aggregates had an average particle size of 94 nm and a variation coefficient of 0.13.

### 3. [Example 4]

### (1) Synthesis of CdSe/ZnS core/shell semiconductor nanoparticles

Synthesis of CdSe/ZnS core/shell semiconductor nanoparticles were carried out as follows.

In Ar gas flow, 2.9 g of stearic acid, 620 mg of n-tetradecylphosphonic acid ofstearic acid, 250 mg of cadmium oxide were added to 7.5 g of tri-n-octylphosphine oxide (TOPO), and then heated and mixed at 370 °C. After a resultant mixture was cooled down to 270 °C, a solvent in which 200 mg of selenium was dissolved in 2.5 ml of tributylphosphine was added thereto, and a resultant mixture was dried under reduced pressure, and CdSe core semiconductor nanoparticles coated with TOPO were obtained.

For synthesis of CdSe/ZnS core/shell semiconductor nanoparticles, 15 g of TOPO was added to the obtained CdSe core particles and heated, and then a solution in which 1.1 g of zinc diethyldithiocarbamate was dissolved in 10 ml of trioctylphosphin was added at 270 °C. Thus, CdSe/ZnS core/shell semiconductor nanoparticles were obtained.

### (2) Formation of semiconductor nanoparticle agglomerate

The aforementioned solution after the reaction was cooled down to a room temperature, and then a 10-fold molar quantity of ethanol serving as a poor solvent relative to the CdSe/ZnS core/shell semiconductor nanoparticles was added to the solution. Thus, CdSe/ZnS agglomerates were obtained.

### (3) Formation of semiconductor nanoparticle aggregate

After repeating centrifugation and ethanol washing of a suspension containing the semiconductor nanoparticle agglomerates for several times, the suspension was replaced with pure water, and an aqueous suspension containing the semiconductor nanoparticle agglomerates were obtained.

After 5 ml of the aqueous suspension went through dispersion for two passes at 150 MPa using a wet type atomizer (Nanomizer produced by Yoshida Kikai Co., Ltd.), 0.4 ml of 10% polyvinyl alcohol aqueous solution and 0.05 ml of 0.5% sodium dodecyl sulfate aqueous solution were added thereto and dispersion thereof was conducted for another three passes.

After a supernatant liquid was removed by centrifugation, a resultant liquid was replaced with pure water, put in the disperser again, and went through dispersion for two passes at 70 Mpa. Thereafter, 0.08 ml of 0.5% sodium tetraborate decahydrate was added thereto, and dispersion was conducted for another three passes. Thus, a dispersion liquid of the semiconductor nanoparticle aggregates was formed. The dispersion liquid obtained was measured by using a particle size distribution measuring device (Zetasizer Nano produced by Malvern Instruments Ltd.), and it was found that the semiconductor nanoparticle aggregates had an average particle size of 161 nm and a variation coefficient of 0.03.

### 4. [Example 5]

### (3) Formation of semiconductor nanoparticle aggregate

After 5 ml of an aqueous suspension containing the semiconductor nanoparticle agglomerates obtained in Example 4 was dispersed for two passes at 150 MPa by using a wet type atomizer (Nanomizer produced by Yoshida Kikai Co. , L.td.), 0.01 ml of mercaptopropyltrimethoxysilane was added thereto and dispersion thereof was conducted for another two passes.

After a supernatant liquid was removed by centrifugation, a resultant liquid was replaced with pure water and ethanol at a ratio between pure water and ethanol of 1:9, put in the disperser again, and dispersed for two passes at 100 Mpa. Thereafter, 0.45 ml of tetraethoxysilane and 0.06 ml of 28% ammonia aqueous solution were added and dispersion was conducted for another three passes. Thus, a dispersion liquid of the semiconductor nanoparticle aggregates was formed. The dispersion liquid obtained was measured by using a particle size distribution measuring device (Zetasizer Nano produced by Malvern Instruments Ltd.), and it was found that the semiconductor nanoparticle aggregates had an average particle size of 173 nm and a variation coefficient of 0.12.

### 5. [Example 7]

### (1) Synthesis of CdTe/ZnS core/shell semiconductor nanoparticles

Synthesis of CdTe/ZnS core/shell semiconductor nanoparticles were carried out following Example 1 described in Japanese Patent Application Laid-open Publication No. 2005-281019.

CdTe core particles were synthesized by following a method set forth in Chemie, volume 100, page 1772 (1996). This means that, under an argon gas atmosphere, hydrogen telluride gas was reacted while vigorously agitating an aqueous solution of cadmium perchlorate prepared to have pH = 11.4 at 25 °C in the presence of thioglycolic acid (HOOCCH2SH) that serves as a surface active agent. By refluxing a resultant aqueous solution for 6 days under an ambient atmosphere, CdTe core particles were obtained. To synthesize CdTe/ZnS core/shell particles, after the aqueous solution was heated to 80 °C, zinc stearate + sulfur dissolved in 1 ml of water were added to the solution so that a ratio among Cd, Te, Zn, and S, that is In/P/Zn/S, became 1/1/1/1. A temperature of a resultant solution was increased from 80 °C to 230 °C to cause reaction for 30 minutes, and CdTe/ZnS core/shell particles were obtained.

### (2) Formation of semiconductor nanoparticle agglomerate

The aforementioned solution after the reaction was cooled down to a room temperature, and then a 10-fold molar quantity of ethanol serving as a poor solvent relative to the CdTe/ZnS core/shell semiconductor nanoparticles was added to the solution. Thus, CdTe/ZnS agglomerates were obtained.

### (3)Formation of semiconductor nanoparticle aggregate

After repeating centrifugation and ethanol washing of a suspension containing the semiconductor nanoparticle agglomerates for several times, the suspension was replaced with pure water, and an aqueous suspension containing the semiconductor nanoparticle agglomerates were obtained.

After 5 ml of the aqueous suspension went through dispersion for five passes at 150 MPa using a wet type atomizer (Nanomizer produced by Yoshida Kikai Co., Ltd.), 0.7 ml of 10% polyvinyl alcohol aqueous solution and 0.12 ml of 0.5% sodium dodecyl sulfate aqueous solution were added thereto and dispersion thereof was conducted for another three passes.

After a supernatant liquid was removed by centrifugation, a resultant liquid was replaced with pure water, put in the disperser again, and went through dispersion for two passes at 110 Mpa. Thereafter, 0.15 ml of 0.5% sodium tetraborate decahydrate was added to the liquid, and dispersion was conducted for another three passes. Thus, a dispersion liquid of the semiconductor nanoparticle aggregates was formed. The dispersion liquid obtained was measured by using a particle size distribution measuring device (Zetasizer Nano produced by Malvern Instruments Ltd.), and it was found that the semiconductor nanoparticle aggregates had an average particle size of 42 nm and a variation coefficient of 0.11.

### 6. [Example 8]

### (3) Formation of semiconductor nanoparticle aggregate

After 5 ml of an aqueous suspension containing the semiconductor nanoparticle agglomerates obtained in Example 7 was dispersed for five passes at 150 MPa by using a wet type atomizer (Nanomizer produced by Yoshida Kikai Co., Ltd.), 0.2 ml of mercaptopropyltrimethoxysilane was added thereto and dispersion thereof was conducted for another three passes.

After a supernatant liquid was removed by centrifugation, a resultant liquid was replaced with pure water and ethanol at a ratio between pure water and ethanol of 1:9, put in the disperser again, and dispersed for two passes at 100 Mpa. Thereafter, 0.65 ml of tetraethoxysilane and 0.14 ml of 28% ammonia aqueous solution were added and dispersion was conducted for another three passes. Thus, a dispersion liquid of the semiconductor nanoparticle aggregates was formed. The dispersion liquid obtained was measured by using a particle size distribution measuring device (Zetasizer Nano produced by Malvern Instruments Ltd.), and it was found that the semiconductor nanoparticle aggregates had an average particle size of 58 nm and a variation coefficient of 0.20.

### 7. [Examples 3, 6, and 9]

Following Example 1 set forth in Japanese Patent Application Laid-open Publication No. 2005-281019, semiconductor nanoparticle aggregates in which InP/ZnS, CdSe/ZnS, and CdTe/ZnS are present in silica matrix were formed.

A dispersion liquid of InP/ZnS, CdSe/ZnS, and CdTe/ZnS core/shell semiconductor nanoparticles was solubilized in water by adding thioglycolic acid thereto as a surface active agent under the conditions at 25 °C and pH = 10. Thereafter, 1.1115 g of a surface active agent, bis (2-ethylhexyl) sulfosuccinate sodium salt (Aerosol OT)(also referred to as "AOT") which is necessary to form reverse micellar (reverse microemersion), was dissolved in 25 ml of isooctane (2, 2, 4-trymethylpentane) serving as a hydrophobic organic solvent, and then, while agitating a resultant solution, 0.74 ml of water, 0.3 ml of the aforementioned water-solubilized InP/ZnS, CdSe/ZnS, and CdTe/ZnS core/shell semiconductor nanoparticle solution was added and dissolved, respectively. Next, while agitating the solution, 0.399 ml of tetraethoxysilane (TEOS) which is alkoxide, and 0.079 ml of 3-aminopropylsilane (APS) which is organic alkoxysilane were added.

By agitating the dispersion liquids for two days, semiconductor nanoparticle aggregates in which InP/ZnS, CdSe/ZnS, and CdTe/ZnS are present in silica matrix were formed.

Results of measurements of luminance and particle sizes of the respective types of semiconductor nanoparticle aggregates obtained as above are collectively shown in Table 1.

Luminance was measured by using a 14 6 nm vacuum ultraviolet lamp (produced by Ushio, Inc.) as a light source, where a sample was set within a vacuum chamber and irradiated from a given distance with a degree of vacuum of 1.33 x 10 Pa, and excited luminescence was measured by a luminance meter. Values of luminescence are expressed as relative values when the InP core particle in Example 1 is defined as 1.

**[Table 1]**

| EXAMPLE No. | SEMICONDUCTOR NANOPARTICLES | COATING MATERIAL | AVERAGE PARTICLE SIZE [NM] | VARIATION COEFFICIENT [%] | LUMINANCE OF AGGREGATE | REMARKS |
|---|---|---|---|---|---|---|
| 1 | InP/ZnS | PVA | 80 | 0.07 | 7800 | PRESENT INTENTION |
| 2 | InP/ZnS | SiO₂ | 94 | 0.13 | 10000 | COMPARATIVE EXAMPLE |
| 3 | InP/ZnS | SiO₂ | 105 | 0.31 | 3800 | COMPARATIVE EXAMPLE |
| 4 | CdSe/ZnS | PVA | 161 | 0.03 | 8200 | PRESENT INVENTION |
| 5 | CdSe/ZnS | SiO₂ | 173 | 0.12 | 300000 | COMPARATIVE EXAMPLE |
| 6 | CdSe/ZnS | SiO₂ | 90 | 0.37 | 7500 | COMPARATIVE EXAMPLE |
| 7 | CdTe/ZnS | PVA | 42 | 0.11 | 4800 | PRESENT INVENTION |
| 8 | CdTe/ZnS | SiO₂ | 58 | 0.2 | 58000 | COMPARATIVE EXAMPLE |
| 9 | CdTe/ZnS | SiO₂ | 100 | 0.41 | 4000 | COMPARATIVE EXAMPLE |

As evident from the results shown in Table 1, the semiconductor nanoparticle aggregate according to the present invention has higher light emission luminance and a smaller variation coefficient of particle size compared to the comparative examples.

## Claims

1. A semiconductor nanoparticle aggregate containing semiconductor nanoparticles having a core/shell structure,
wherein an agglomeration state of an agglomerate made by agglomerating the semiconductor nanoparticles is controlled by using physical energy, thereby forming the semiconductor nanoparticle aggregate;
wherein a coating structure is formed so as to coat the agglomerate made by agglomerating the semiconductor nanoparticles, thereby forming the semiconductor nanoparticle aggregate;
wherein a raw material that configures the coating structure is hydrophilic polymer; wherein an average particle size of the nanoparticle aggregate is in a range from 30 to 300 nm;
**characterized in that** a variation coefficient of particle size of the nanoparticle aggregate is in a range from 0.02 to 0.2 and wherein the particle sizes of the semiconductor nanoparticle aggregates are measured by a dynamic light scattering method.

2. The semiconductor nanoparticle aggregate according to claim 1, wherein the raw material that forms the coating structure is polyvinyl alcohol.

3. The semiconductor nanoparticle aggregate according to claim 1 or 2, wherein a raw material that forms a shell part of the semiconductor nanoparticle having the core/shell structure is zinc sulfide (ZnS) or silicon dioxide (SiO₂).

4. The semiconductor nanoparticle aggregate according to any one of claims 1 to 3, wherein a raw material that forms a core part of the semiconductor nanoparticle having the core/shell structure is selected from Si, Ge, InN, InP, GaAs, AlSe, CdSe, AlAs, GaP, ZnTe, CdTe, and InAs.

5. The semiconductor nanoparticle aggregate according to any one of claims 1 to 4, further comprising a surface modifier on a surface of the semiconductor nanoparticle aggregate, wherein the surface modifier is mercaptopropionic acid, mercaptoundecanoic acid, or aminopropanethiol.

6. The semiconductor nanoparticle aggregate according to any one of claims 1 to 5, wherein a disperser is used to supply the physical energy and an amount of physical energy is 20 MPa to 300 MPa.

7. The semiconductor nanoparticle aggregate according to any one of claim 6, wherein the amount of physical energy is 70 MPa to 150 MPa.

8. A production method for a semiconductor nanoparticle aggregate containing semiconductor nanoparticles having a core/shell structure, comprising:
controlling by using physical energy an agglomeration state of an agglomerate made by agglomerating the semiconductor nanoparticles, thereby forming the semiconductor nanoparticle aggregate;
wherein a coating structure is formed so as to coat the agglomerate made by agglomerating the semiconductor nanoparticles, thereby forming the semiconductor nanoparticle aggregate;
wherein a raw material that configures the coating structure is hydrophilic polymer;
wherein an average particle size of the nanoparticle aggregate is in a range from 30 to 300 nm;
**characterized in that** a variation coefficient of particle size of the nanoparticle aggregate is in a range from 0.02 to 0.2 and wherein the particle sizes of the semiconductor nanoparticle aggregates are measured by a dynamic light scattering method.

9. The method according to claim 8, further comprising binding a surface modifier to a surface of the semiconductor nanoparticle aggregate, wherein the surface modifier is mercaptopropionic acid, mercaptoundecanoic acid, or aminopropanethiol.

10. The method according to claim 8 or 9, wherein a disperser is used to supply the physical energy and an amount of physical energy is 20 MPa to 300 MPa.

11. The method according to claim 10, wherein the amount of physical energy is 70 MPa to 150 MPa.

## Patentansprüche

1. Halbleiter-Nanopartikel-Aggregat enthaltend Halbleiter-Nanopartikel mit einer Kern/Schalen-Struktur,
wobei ein Agglomerationszustand eines durch Agglomeration der Halbleiter-Nanopartikel hergestellten Agglomerats unter Verwendung von physikalischer Energie kontrolliert wird, und dadurch Bilden des Halbleiter-Nanopartikel-Aggregats;
wobei eine Beschichtungsstruktur gebildet wird, so dass das durch Agglomeration der Halbleiter-Nanopartikel hergestellte Agglomerat beschichtet und so das Halbleiter-Nanopartikel-Aggregat gebildet wird;
wobei ein Rohmaterial, das die Beschichtungsstruktur bildet, ein hydrophiles Polymer ist;
wobei eine mittlere Teilchengröße des Nanopartikel-Aggregats im Bereich von 30 bis 300 nm liegt;
**dadurch gekennzeichnet, dass** ein Teilchengröße-Variationskoeffizient des Nanopartikel-Aggregats in einem Bereich von 0,02 bis 0,2 liegt und wobei die Teilchengrößen der Halbleiter-Nanopartikel-Aggregate durch ein dynamisches Lichtstreuverfahren gemessen werden.

2. Halbleiter-Nanopartikel-Aggregat nach Anspruch 1, wobei das Rohmaterial, das die Beschichtungsstruktur bildet, Polyvinylalkohol ist.

3. Halbleiter-Nanopartikel-Aggregat nach Anspruch 1 oder 2, wobei ein Rohmaterial, das einen Schalenteil des Halbleiter-Nanopartikels mit der Kern/Schalen-Struktur bildet, Zinksulfid (ZnS) oder Siliciumdioxid (SiO₂) ist.

4. Halbleiter-Nanopartikel-Aggregat nach einem der Ansprüche 1 bis 3, wobei eine Rohmaterial, das einen Kernteil des Halbleiter-Nanopartikels mit Kern/Schalen-Struktur bildet, ausgewählt ist aus Si, Ge, InN, InP, GaAs, AlSe, CdSe, AlAs, GaP, ZnTe, CdTe und InAs.

5. Halbleiter-Nanopartikel-Aggregat nach einem der Ansprüche 1 bis 4, weiterhin umfassend einen Oberflächen-Modifizierer auf einer Oberfläche des Halbleiter-Nanopartikel-Aggregats, wobei der Oberflächen-Modifizierer Mercaptopropionsäure, Mercaptoundecansäure oder Aminopropanthiol ist.

6. Halbleiter-Nanopartikel-Aggregat nach einem der Ansprüche 1 bis 5, wobei ein Dispergator verwendet wird, um die physikalische Energie bereitzustellen und eine Menge an physikalischer Energie von 20 bis 300 MPa beträgt.

7. Halbleiter-Nanopartikel-Aggregat nach einem Anspruch 6, wobei die Menge an physikalischer Energie 70 bis 150 MPa beträgt.

8. Verfahren zur Herstellung eines Halbleiter-Nanopartikel-Aggregats enthaltend Halbleiter-Nanopartikel mit einer Kern/Schalen-Struktur, umfassend:
Kontrollieren unter Verwendung der physikalischen Energie eines Agglomerationszustands eines durch Agglomeration der Halbleiter-Nanopartikel hergestellten Agglomerats und dadurch Bilden des Halbleiter-Nanopartikel-Aggregats;
wobei ein Rohmaterial, das die Beschichtungsstruktur bildet, ein hydrophiles Polymer ist;
wobei eine mittlere Teilchengröße des Nanopartikel-Aggregats im Bereich von 30 bis 300 nm liegt;
**dadurch gekennzeichnet, dass** ein Teilchengröße-Variationskoeffizient des Nanopartikel-Aggregats in einem Bereich von 0,02 bis 0,2 liegt und wobei die Teilchengrößen der Halbleiter-Nanopartikel-Aggregate durch ein dynamisches Lichtstreuverfahren gemessen werden.

9. Verfahren nach Anspruch 8, weiterhin umfassend das Binden eines Oberflächen-Modifizierer an eine Oberfläche des Halbleiter-Nanopartikel-Aggregats, wobei der Oberflächen-Modifizierer Mercaptopropionsäure, Mercaptoundecansäure oder Aminopropanthiol ist.

10. Verfahren nach Anspruch 8 oder 9, wobei ein Dispergator verwendet wird, um die physikalische Energie bereitzustellen und eine Menge an physikalischer Energie von 20 bis 300 MPa beträgt.

11. Verfahren nach Anspruch 10, wobei die Menge an physikalischer Energie von 70 bis 150 MPa beträgt.

## Revendications

1. Agrégat de nanoparticules semiconductrices contenant des nanoparticules semiconductrices ayant une structure coeur/coque,
dans lequel un état d'agglomération d'un agglomérat fabriqué par l'agglomération des nanoparticules semiconductrices est contrôlé en utilisant de l'énergie physique, formant ainsi l'agrégat de nanoparticules semiconductrices ; dans lequel une structure de revêtement est formée pour revêtir l'agglomérat fabriqué par l'agglomération des nanoparticules de semi-conducteur, formant ainsi l'agrégat de nanoparticules semiconductrices ;
dans lequel une matière brute qui configure la structure de revêtement est un polymère hydrophile ;
dans lequel une taille de particule moyenne de l'agrégat de nanoparticules se situe dans la plage allant de 30 à 300 nm ;
**caractérisé en ce qu'**un coefficient de variation de taille de particule de l'agrégat de nanoparticules se situe dans une plage de 0,02 à 0,2 et dans lequel les tailles de particule des agrégats de nanoparticules du semi-conducteur sont mesurées par un procédé de diffusion de lumière dynamique.

2. Agrégat de nanoparticules semiconductrices selon la revendication 1, dans lequel la matière brute qui forme la structure de revêtement est l'alcool polyvinylique.

3. Agrégat de nanoparticules semiconductrices selon la revendication 1 ou 2, dans lequel une matière brute qui forme une partie de coque de la nanoparticule semiconductrices ayant la structure coeur/coque est le sulfure de zinc (ZnS) ou le dioxyde de silicium (SiO₂).

4. Agrégat de nanoparticules semiconductrices selon l'une quelconque des revendications 1 à 3, dans lequel une matière brute qui forme une partie de coeur de la nanoparticule semiconductrices ayant la structure coeur/coque est choisie parmi Si, Ge, InN, InP, GaAs, AlSE, CdSe, AlAs, GaP, ZnTe, CdTe et InAs.

5. Agrégat de nanoparticules semiconductrices selon l'une quelconque des revendications 1 à 4, comprenant en outre un modificateur de surface sur une surface de l'agrégat de nanoparticules de semi-conducteur, dans lequel le modificateur de surface est l'acide mercaptopropionique, l'acide mercaptoundécanoïque ou l'aminopropanethiol.

6. Agrégat de nanoparticules semiconductrices selon l'une quelconque des revendications 1 à 5, dans lequel un appareil de dispersion est utilisé pour fournir l'énergie physique et une quantité d'énergie physique est de 20 à 300 MPa.

7. Agrégat de nanoparticules semiconductrices selon l'une quelconque de la revendication 6, dans lequel la quantité d'énergie physique est de 70 à 150 MPa.

8. Procédé de production d'un agrégat de nanoparticules semiconductrices contenant des nanoparticules semiconductrices ayant une structure coeur/coque, comprenant :
le contrôle en utilisant de l'énergie physique d'un état d'agglomération d'un agglomérat fabriqué par l'agglomération des nanoparticules de semi-conducteur, formant ainsi l'agrégat de nanoparticules semiconductrices ;
dans lequel une structure de revêtement est formée pour revêtir l'agglomérat fabriqué par l'agglomération des nanoparticules de semi-conducteur, formant ainsi l'agrégat de nanoparticules semiconductrices ;
dans lequel une matière brute qui configure la structure de revêtement est un polymère hydrophile ;
dans lequel une taille de particule moyenne de l'agrégat de nanoparticules se situe dans la plage allant de 30 à 300 nm ;
**caractérisé en ce qu'**un coefficient de variation de taille de particule de l'agrégat de nanoparticules se situe dans une plage de 0,02 à 0,2 et dans lequel les tailles de particule des agrégats de nanoparticules du semi-conducteur sont mesurées par un procédé de diffusion de lumière dynamique.

9. Procédé selon la revendication 8, comprenant en outre la liaison d'un modificateur de surface à une surface de l'agrégat de nanoparticules de semi-conducteur, dans lequel le modificateur de surface est l'acide mercaptopropionique, l'acide mercaptoundécanoïque ou l'aminopropanethiol.

10. Procédé selon la revendication 8 ou 9, dans lequel un appareil de dispersion est utilisé pour fournir l'énergie physique et une quantité d'énergie physique est de 20 à 300 MPa.

11. Procédé selon la revendication 10, dans lequel la quantité d'énergie physique est de 70 à 150 MPa.
